# FASCICULE DE BREVET EUROPEEN

(11) **EP 1 075 180 B1**
(45) Date de publication et mention de la délivrance du brevet: **15.10.2003**
(21) Numéro de dépôt: 99916953.5
(22) Date de dépôt: 29.04.1999
(51) Int. Cl.: A01K 61/00, A01K 67/02

(54) **PROCEDE DE REPRODUCTION ET D'ELEVAGE EN MILIEU AQUATIQUE NOTAMMENT DE SILURUS GLANIS ET DISPOSITIF PERMETTANT DE LE METTRE EN OEUVRE**
VERFAHREN ZUM ZÜCHTEN VON SILURUS GLANIS IN EINER WÄSSRIGEN UMGEBUNG UND VORRICHTUNG ZUR DURCHFÜHRUNG DIESES VERFAHRENS
METHOD FOR REPRODUCING AND REARING SILURUS GLANIS IN PARTICULAR IN AQUATIC MEDIUM AND IMPLEMENTING DEVICE

(30) Priorité: 30.04.1998 FR 9805546; 31.12.1998 FR 9816759
(43) Date de publication de la demande: 14.02.2001
(60) Demande divisionnaire: 03075348.7
(73) Titulaire: Ribes, André, 34530 Montagnac (FR); Ribes, Gilbert, 34530 Montagnac (FR)
(72) Inventeur: Ribes, André, 34530 Montagnac (FR); Ribes, Gilbert, 34530 Montagnac (FR)
(74) Mandataire: Richebourg, Michel François
(86) Numéro de dépôt international: FR9901017
(87) Numéro de publication internationale: WO99056534

(56) Documents cités:
- FR-A- 2 187 214
- US-A- 3 765 372
- US-A- 4 182 269
- US-A- 4 214 551
- US-A- 4 489 674
- US-A- 4 697 546
- US-A- 4 742 798

## Description

La présente invention concerne les domaines de l'aquaculture et de la pisciculture et notamment les adaptations et les moyens qui rendent possibles la reproduction et l'élevage, dans des conditions optimales des différentes espèces vivant en milieux aquatiques comme par exemple les poissons, crustacés, mollusques, etc...

Les insuffisances graves des modes de production aquacoles et piscicoles, tels que pratiqués actuellement, sont bien connues. Elles se traduisent par des- difficultés aux plans de la qualité et de l'image des productions et ont des conséquences négatives au plan de leur commercialisation. Elles se manifestent principalement au niveau des conduites d'élevage et de la gestion des biotopes correspondants souvent conçues et réalisées, pour la recherche d'une productivité accrue, en contradiction avec les exigences fondamentales de la biologie.

Les contraintes et les dérives résultant de l'intensification en matière de productions aquacoles et piscicoles conduisent couramnent à confiner - avec des -densités importantes - les sujets élevés dans des bassins de type artificiel- en béton ou en matières "plastiques", etc...- ou bien dans des cages immergées en étangs ou en mer, les privant ainsi du contact normal avec l'adversité naturelle que doit affronter l'espèce concernée, notamment aux niveaux bactériens, parasitaires, etc..

La mise en place et le développement des défenses immunitaires s'en trouvent fortement perturbés ou inhibés chez les sujets concernés, créant ainsi une situation de dépendance par rapport aux moyens de la pharmacie vétérinaire et plus particulièrement, aux antibiotiques souvent incorporés directement lors de leur fabrication dans la composition des aliments habituellement distribués.

Cette situation de dépendance est, de surcroît, aggravée par le recours systématique à une alimentation entièrement artificielle dont la production industrielle ne peut' assurer une parfaite continuité des données qualitatives, ni éviter, de ce fait, l'apparition de carences souvent à l'origine des défaillances du système immunitaire ou de leur aggravation.

Ces aspects négatifs des formes conventionnelles, et notamment intensives, d'élevage aquacole et piscicole régulièrement constatés dans les phases de grossissement des alevins et des juvéniles vers leurs tailles commerciales prennent une signification beaucoup plus importante encore, par leurs conséquences, dans les phases de reproduction et d'élevage larvaire.

En effet, les gamètes de géniteurs retenues parmi les sujets élevés suivant les modes conventionnels décrits antérieurement n'ont pas le niveau élevé de qualité biologique des gamètes de géniteurs vivant dans les conditions naturelles ou dans des conditions de type naturel, notamment au niveau du vitellus des ovocytes.

L'embryogénèse et la vie larvaire en sont fortement affectées conduisant, dans cette situation également, à un recours très précoce, c'est à dire dès la phase d'incubation, aux moyens de la pharmacie vétérinaire. Cette situation entraîne une fragilisation des nouveaux sujets, initiée dès l'oeuf et l'embryon, créant ainsi les conditions d'une déficience définitive.

En outre, certains aspects des protocoles de reproduction couramment employés notamment pour les poissons d'eau douce accentuent encore l'état initial biologiquement déficient de l'embryon et de la larve.

En effet, à l'occasion des manipulations de fécondation et d'incubation, pour éviter que les oeufs ne se collent entre eux et ne s'agglutinent -ce qu'ils feraient naturellement mais les conduirait pour la plupart à périr par défaut d'oxygénation- il est pratiqué leur "décollage" au moyen, notamment, d'enzymes protéolytiques additionnés à l'eau de l'incubateur.

Ces produits cumulent leurs effets avec ceux des fongicides, anti-parasitaires, bactéricides, utilisés couramment d'une manière conventionnelle en cours d'incubation. Il en résulte une détérioration plus ou moins importante suivant les doses employées de la membrane des oeufs, décollés et en mouvement dans l'incubateur, au moment où son rôle notamment dans l'oxygénation de l'embryon doit être, au contraire, favorisé.

En outre, les oeufs décollés flottent dans l'incubateur où ils sont inutilement en continuelle agitation; leur contrôle, comme par exemple l'observation de l'embryogénèse en cours, est alors très difficile sinon impossible.

Par contre en aquaculture semi-artificielle, il est parfois prévu de placer dans les zones de frai, des nids confectionnées en branchages afin de récupérer les oeufs; nids pouvant être ensuite placés dans les incubateurs.

Une des méthodes ayant pour but de potentialiser les résultats de l'aquaculture, notamment en matière de croissance des sujets élevés pour leur commercialisation, consiste précisément à rendre impossible les activités de reproduction et donc d'empêcher les maturations successives après la puberté.

Un des moyens permettant de rendre impossibles les activités de reproduction des sujets élevés, obtenu pour les salmonidés, est de les rendre triploïdes.

La triploïdie qui existe naturellement de manière exceptionnelle dans les milieux aquatiques correspond à la présence dans le noyau de la cellule de trois lots chromosomiques identiques -chacun représentant le patrimoine génétique du sujet- au lieu des deux lots chromosomiques identiques qui définissent la diploïdie, situation normale de la cellule.

La triploïdie n'est pas une manipulation génétique; le lot chromosomique représentant le patrimoine génétique n'est pas affecté et reste identique qu'un même sujet soit triploïde ou diploïde. Le sujet triploïde est donc normalement sexué mais il est stérile.

En l'absence d'activité de reproduction, la totalité de l'énergie consommée par le sujet élevé sera consacrée à sa croissance qui sera donc beaucoup plus rapide que celle du même sujet diploïde : la productivité de l'aquaculture s'en trouve fortement accrue.

La triploïdie permet également, par la stérilité qui en résulte, de maîtriser totalement la démographie de l'espèce concernée; elle rend ainsi possible son introduction dans le milieu naturel notamment à des fins écologiques, sans courir le risque de perdre le contrôle de sa population et d'une prolifération dangereuse; cela prend une signification particulière lorsqu'il s'agit, par exemple, d'un carnassier de très grande taille, comme Silurus Glanis.

En outre, la triploïdie supprime les risques de pollution génétique et autorise l'utilisation de l'espèce concernée à des fins écologiques ou d'élevage aquacole dans des zones où elle n'est pas naturellement présente et où elle ne fait donc pas partie des écosystèmes existants. Dans toutes ces situations, les nécessités de la lutte pour la préservation des espèces naturelles et pour la sauvegarde des écosystèmes conduisent à des attitudes administratives et à des réglementations interdisant l'introduction et l'utilisation des sujets diploïdes de l'espèce concernée étrangère. Dans le cas de Silurus Glanis par exemple, naturellement présent dans les parties continentales de la zone tempérée en Europe et en Asie occidentale, la triploïdie de l'espèce en permet l'introduction et l'utilisation, techniquement et administrativement, dans d'autres zones climatiques, par exemple tempérées chaudes, subtropicales, tropicales, équatoriales, etc...

Une des méthodes connues pour obtenir la triploïdie consiste à appliquer un choc thermique à l'oeuf; ce choc thermique doit être réalisé dans des conditions particulières, à un moment précis, à une température donnée et durant un temps déterminé.

Une des conséquences du choc thermique est de détériorer relativement l'oeuf traité notamment au niveau du vitellus et d'avoir, de ce fait, un effet négatif sur l'embryogénèse.

Les conditions de maturation, de reproduction, d'incubation en aquaculture conventionnelle amplifient considérablement ces difficultés, jusqu'à rendre la triploïdie inaccessible pour la plupart des espèces commerciales - si l'on excepte les salmonidés - et à ne l'obtenir qu'avec des taux de triploïdie aléatoires par rapport aux sujets traités.

C'est donc dans une sorte de spirale négative que les insuffisances de l'activité aquacole et piscicole conventionnelle se constatent en se renforçant mutuellement tant au niveau des phases de grossissement des alevins et des juvéniles vers leurs tailles commerciales qu'au niveau de la génération -maturations des géniteurs, reproduction, élevage larvaire- des futurs sujets destinés au grossissement et à la poursuite de la génération elle-même.

Un exemple de procédé d'élevage et de reproduction en milieu aquatique est décrit dans le brevet américain n° 3,765,372 qui dispose que pour chaque phase classique de l'élevage aquatique, à savoir
- éclosion, résorption vésiculaire, différents stades de l'élevage larvaire, élevage jusqu'au stade d'alevin ou de juvénile en écloserie,
- croissance et grossissement en bassins,
- passagé de la phase alevin ou de la phase juvénile à la phase adulte,
- sélection des géniteurs, maturation,
- prélèvement des ovocytes et des spermatozoïdes,
- fécondation,
- embryogénèse en incubateur,
la température est adaptée, c'est à dire qu'une température optimale est définie pour quasiment chaque phase d'élevage et que les moyens de son contrôle soit la font évoluer dans un seul et même bassin soit correspondent au déplacement de bassins en bassins - où l'eau est maintenue à la température optimale définie pour la phase correspondante de leur élevage - des oeufs, larves juvéniles, etc...

Ainsi, bien que cela n'apparaisse pas directement du fait d'un exemple de réalisation basé sur une espèce pour laquelle la température de l'environnement évolue peu, le procédé décrit dans ce document reproduit artificiellement l'évolution naturelle saisonnière des températures de l'eau au fur et à mesure de l'évolution des sujets élevés.

La mise en oeuvre de moyens artificiels contribue simplement à créer pour chaque phase un maintien régulier et sûr de la température et de la luminosité afin de correspondre le plus possible mais sans les aléas et variations naturelles, à la température et à la luminosité optimales auxquelles sont confrontés en milieu naturel, l'oeuf, la larve le juvénile ou le poisson lors d'une phase donnée. Ce procédé artificiel peut éventuellement permettre de décaler les pontes par rapport au cycle naturel.

D'une façon générale, ce document décrit un procédé d'élevage dans lequel est impliqué un dispositif global où les variations et les modulations notamment de la température et de l'éclairage sont sollicitées pour obtenir des résultats biologiques. Ces variations et ces modulations tendent à retrouver les incitateurs naturels que l'espèce connaît et rencontre périodiquement dans son biotope naturel. Ces modulations sont en phase avec les dits incitateurs et non en contradiction avec eux. Aussi, l'espèce réagit naturellement à leur présence ou à leur absence, reconnues par leur nature (augmentation ou diminution d'un paramètre) ou par leur intensité (niveau de la variation du ou des paramètres concernés).

Ce document ne fait pas mention d'un support d'oeufs susceptible de réaliser le déplacement de ces derniers. Il décrit toutefois, la possibilité de récupérer à la surface de l'incubateur les oeufs flottants et d'évacuer du fond de l'incubateur les oeufs morts. Il n'est pas non plus décrit que les oeufs sont déplacés à partir de l'incubateur.

Le document américain n° 4,742,798 au nom de Blackett décrit quant à lui, un incubateur adoptant une plaque percée statique, disposée à mi-hauteur sur laquelle peuvent être déposés des substrats support d'oeufs également ajourés de façon à non seulement servir de supports à des oeufs non collants déposés à leur surface mais également favoriser leur oxygénation facilitant par ce fait, leur incubation. Néanmoins, les substrats sont constitués par de nombreuses formes alvéolées indépendantes les unes des autres susceptibles de servir de supports à un ou plusieurs oeufs mais ne pouvant pas faire l'objet d'une amovibilité globale, c'est à dire synchronisée pour l'ensemble des substrats. Ces substrats qui sont destinés à plus ou moins reproduire le support sur lequel est susceptible de pondre certaines espèces de poissons en milieu naturel tels les galets, reposent de par leur poids sur la plaque percée. De plus, la plaque percée permettant de réguler le flux de liquide à l'intérieur de l'incubateur n'est pas conçue pour être retirée et replacée en position. Il est même prévu que cette plaque soit soudée'aux bords de l'incubateur.

La présente invention apporte une solution aux insuffisances de l'art ancien (ou à la pratique conventionnelle) principalement sur les points décrits. Elle y parvient en évitant ou en supprimant les contradictions entre les procédés d'élevage et les contraintes naturelles ordinaires de la biologie, et au contraire en développant, en normalisant et en potentialisant les moyens naturels dont la nature ne dispose souvent que d'une manière discontinue et/ou aléatoire.

Une des contraintes naturelles de la biologie des animaux des milieux aquatiques de la zone tempérée réside précisément dans le fait qu'en situation naturelle, ils n'ont d'activité biologique, et notamment de croissance, que saisonnièrement pendant une période donnée de l'année, à savoir lorsque la température de l'eau a atteint, puis dépassé, un certain seuil thermique, différent pour chaque espèce. Au-dessus de ce seuil, l'activité biologique, notamment la croissance, s'accroît avec l'élévation de la température, jusqu'à devenir maximale pour une température donnée, différente pour chaque espèce. Cette température correspond à l'optimum thermique biologique de l'espèce.

Aussi, un des objectifs de l'invention est non seulement de libérer l'élevage des animaux des milieux aquatiques de la zone tempérée de la contrainte d'une activité biologique et notamment d'une croissance optimales réduites à une seule période de l'année mais aussi, de dépasser le stade d'une simple reproduction par des moyens artificiels de la variation saisonnière des températures de l'eau suivant. les différentes phases classiques d'évolution et d'élevage des sujets.

Le procédé de reproduction et d'élevage de l'invention sur la base d'oeufs susceptibles d'être fixés est du type de celui comprenant les phases classiques suivantes :
- éclosion, résorption vésiculaire, différents stades de l'élevage larvaire, élevage jusqu'au stade d'alevin ou de juvénile en écloserie,
- croissance et grossissement en bassins,
- passage de la phase alevin ou de la phase juvénile à la phase adulte,
- sélection des géniteurs, maturation,
- prélèvement des ovocytes et des spermatozoïdes,
- fécondation,
- embryogénèse en incubateur.

Selon la caractéristique principale de l'invention, le procédé de reproduction et d'élevage est remarquable en ce qu'il consiste à maintenir constamment la température de l'eau pendant l'ensemble des phases classiques d'élevage, à une même température unique la plus proche possible de l'optimum thermique biologique de l'espèce élevée.

Le maintien à une température proche de l'optimum thermique biologique permet de mettre en oeuvre, en continu, des taux de croissance très élevés des espèces propres aux milieux aquatiques de la zone tempérée, espèces qui, par nature dans cette zone, ne sont biologiquement actives et n'ont de croissance que d'une manière saisonnière très discontinue au-dessus d'un certain seuil thermique propre à chaque espèce.

Le maintien à une température proche de l'optimum thermique biologique permet également de parvenir à supprimer la formation de réserves lipidiques nécessaires à la survie des espèces vivant dans les milieux aquatiques de la zone tempérée pendant la période d'inactivité biologique; la qualité de la chair devenant essentiellement protéique et sa valeur commerciale en sont considérablement améliorées.

Les avantages d'une aquaculture à une température optimale constante pour l'ensemble de l'élevage sont donc quantitatifs et qualitatifs.

A l'opposé du procédé décrit dans le document américain n°US-A-3 765 372, dans le nouveau cycle tel que le définissent les demandeurs, l'optimum thermique biologique appliqué invariablement et en continu à une espèce et plus particulièrement à une espèce de la zone tempérée crée pour cette espèce une situation environnementale nouvelle ou nouveau biotope complètement étranger à celle qu'elle connaît en milieu naturel au plan thermique. Les fonctions biologiques -croissance, reproduction, etc...- se réaliseront donc différemment, dans le cadre de cette situation environnementale elle-même différente, (caractérisé notamment par l'optimum thermique biologique correspondant au taux de croissance optimum appliqué de manière homogène et constante; à l'opposé d'une procédé où la température, notamment, est adapcée pour chaque phase biologique en fonction des situations naturelles correspondantes ou homologues.). En outre, les aléas provoqués par la maturation sexuelle, sont complètement contrôlés et évités par la triploïdie qui fait l'objet d'une autre revendication.

Lorsque l'animal aquatique élevé est Silurus Glanis, le procédé est remarquable en ce qu'il consiste à maintenir constamment la température de l'eau pendant l'ensemble des phases classiques d'élevage, à une même température unique la plus proche possible de 26° celcius.

Ainsi, l'élevage optimisé de Silurus Glanis nécessite un maintien de température à l'optimum thermique biologique sur l'ensemble des phases classiques d'élevage comprenant notamment l'embryogénèse.

Selon un mode de réalisation s'adaptant à des espèces ne pouvant supporter l'optimum thermique biologique sur l'ensemble des phases, le procédé est remarquable en ce qu'il consiste à appliquer une température inférieure à celle correspondante à l'optimum thermique biologique pour les phases d'embryogénèse en incubateur et de résorption vésiculaire.

L'obtention de cette température optimale peut être réalisée de différentes manières non limitatives. Ainsi, par exemple, l'aquaculture selon l'invention pourra être pratiquée en zone tropicale ou en zone équatoriale où les températures optimales constantes recherchées des milieux aquatiques sont naturelles. Mais elles pourront également être obtenues à partir de nappes phréatiques chaudes, de ressources géothermiques, ou bien artificiellement par des moyens conventionnels de chauffage de l'eau. Ainsi, le maintien de la température optimale constante n'est pas dépendant du lieu d'implantation de l'aquaculture.

Bien entendu, lorsque les bassins naturels se trouvent en zone tropicale, la température dépend des facteurs météorologiques naturels de ladite zone tropicale. Aussi, bien que la température de l'eau y soit naturellement très proche de l'optimum thermique biologique tout au long de l'année - contrairement aux conditions de la zone tempérée - elle ne peut y être rigoureusement constante et est soumise à certaines variations.

Dans le cas de Silurus Glanis qui peut être étendu aux autres espèces vivant en milieux aquatiques, l'invention a recours de préférence aux bassins de type naturel et non aseptisé, qui peuvent être creusés directement, à même le sol, et où pourront se créer des biotopes identiques à ceux que connait l'espèce en milieu naturels ou très proches de ceux-ci; biotopes qu'en aucun cas on ne cherchera à rendre aseptiques. Les demandeurs ne veulent pas néanmoins limiter l'invention à l'exploitation de bassins naturels non aseptisés. Aussi, pour certaines applications, des bassins artificiels, pourront être exploités.

Le procédé de l'invention se distingue du procédé décrit dans l'art antérieur et des procédés classiques d'élevage en maintenant une température d'optimum thermique biologique de l'eau pour l'ensemble des phases classiques de l'élevage d'animaux aquatiques et non en reproduisant optimalement pour chaque phase la température optimale à laquelle est soumise en milieu naturel l'espèce élevée, ladite température étant soumise à variation notamment pour l'élevage d'espèce de la zone tempérée.

Un des moyens originaux de l'invention consiste à assurer l'alimentation en écloserie, en milieu entièrement artificiel, des larves élevées et juvéniles - nés par reproduction artificielle - uniquement sur la base d'aliments artificiels du commerce, en excluant tout aliment vivant.

Cela est rendu possible par la qualité biologique des larves, alevins et juvéniles, elle-même résultant de la qualité biologique des géniteurs mis en oeuvre acquise dans Le cycle biologique- présentement décrit dans le cadre de l'invention- et de l'application correcte d'un protocole de reproduction adéquat.

Les jeunes sujets, alevins ou juvéniles, seront ainsi, à leur sortie de l'écloserie parfaitement conditionnés à l'alimentation artificielle qu'ils continueront de pratiquer quand ils seront alors placés dans des bassins de type naturel. Le sevrage et ses conséquences négatives pour la survie, la croissance et les qualités biologiques sont ainsi supprimés.

Il sera alors nécessaire et possible de mettre à leur disposition une alimentation naturelle complémentaire qu'ils utiliseront en fonction de leur besoins biologiques de complémentation de l'aliment artificiel pour assurer de la sorte parfaitement leur équilibre alimentaire. Ainsi, une des caractéristiques du procédé de l'invention, consiste à assurer l'alimentation en bassins sur la base d'une association d'aliments naturels et d'aliments artificiels.

Dans le cas de Silurus Glanis ou de tout autre espèce carnassière, cette alimentation naturelle complémentaire pourra se présenter sous la forme d'une seconde espèce de "poissons proies" ou autres espèces d'animaux aquatiques vivant et éventuellement se reproduisant dans le même biotope que l'espèce principale qui pourra en exercer la prédation librement précisément en fonction de ses besoins propres de complémentation de l'alimentation artificielle.

Il existe dans l'art antérieur plusieurs cas d'élevage en polyculture, par exemple l'élevage de Silurus Glanis avec des carpes, les carpes constituant une partie de l'alimentation de Silurus Glanis.

Dans le cadre de l'invention, le recours à un biotope à température optimale constante - proche des conditions thermiques tropicales naturelles- pour l'aquaculture d'espèces de la zone tempérée à taux de croissance élevés, tel Silurus Glanis, permet de leur associer, pour leur prédation, des espèces de la zone tropicale dont la reproduction naturelle répétée régulièrement tout au long de l'année assurera la présence en continu de proies aux tailles voulues.

L'association d'une alimentation naturelle à l'alimentation artificielle de base assure la complémentation qualitative de cette dernière et garantit l'équilibre alimentaire et biologique de l'espèce élevée.

Elle peut également permettre la substitution dans l'alimentation artificielle des protéines provenant de "farines d'animaux terrestres", considérées comme dangereuses au plan sanitaire, par des protéines d'origine végétale.

La correspondance recherchée par les demandeurs entre leur procédé d'aquaculture de l'invention et les données naturelles - elles mêmes optimisées- permet de conserver et d'améliorer les avantages décisifs de l'adaptation des espèces à leur milieu naturel parmi lesquels la capacité de régler par leurs propres défenses immunitaires les problèmes et difficultés que peut leur poser leur propre biotope; et également la capacité d'assurer biologiquement les conditions d'une reproduction "efficace et opératoire" de leur espèce. Ainsi, contrairement à l'aquaculture conventionnelle, le procédé de l'invention intègre, en l'optimisant, le cycle de la biologie naturelle.

Cette intégration qui définit un nouveau cycle, à la fois naturel et artificiel, est d'une grande cohérence. Ce cycle permet d'obtenir, dans les meilleures conditions biologiques, d'excellents produits pour leur commercialisation, mais également des géniteurs en conditions optimales qui eux-mêmes rendront possible et pratiquable la triploïdie, notamment de Silurus Glanis, en relativisant considérablement les conséquences du choc thermique.

En outre, les larves nées dans ces conditions, diploïdes ou triploïdes, pourront être élevées jusqu'à la phase alevin ou juvénile avec une alimentation exclusivement artificielle, ré-amorçant ainsi le cycle à la fois artificiel et naturel propre à l'invention.

La sélection des géniteurs parmi les meilleurs sujets mis en évidence dans le cours du cycle lui-même, permet d'en assurer le renouvellement quasi-indéfiniment en obtenant et en "confirmant" une biologie des animaux des milieux aquatiques très proche de celle qui correspond aux données de la biologie naturelle quand les conditions et les opportunités naturelles sont les plus favorables, données elles-mêmes optimisées par les moyens de l'invention.

Ce haut niveau de qualité biologique est absolument différent de celui que l'on observe en aquaculture conventionnelle notamment en aquaculture intensive.

Selon une autre caractéristique particulièrement intéressante de l'invention, le procécé d'aquaculture est remarquable en ce qu'il consiste à placer dans l'incubateur, avant l'introduction des oeufs, un support amovible les accueillant sur des zones de réception permettant aux oeufs de s'y fixer au moment de leur introduction dans l'incubateur, lequel support permet, à un même moment précis, le déplacement de la totalité des oeufs.

Ce support a pour avantage de permettre la fixation individuelle des oeufs qui sont ainsi immobilisés pendant toute la durée de l'embryogénèse chacun étant parfaitement exposé à la circulation de l'eau dans l'incubateur et de ce fait bénéficiant notamment d'un oxygénation optimale.

La fixation individuelle des oeufs sur le support évite qu'ils ne s'agglutinent entre eux et de ce fait périssent pour la plupart par insuffisance d'oxygénation et rend inutile l'addition à l'eau de l'incubateur de produits, tels les enzymes protéolytiques, destinés précisément en aquaculture conventionnelle à les séparer après qu'ils se soient agglutinés.

En outre, les oeufs étant fixés et immobilisés et non plus inutilement en mouvement pendant toute la durée de l'incubation, l'embryogénèse pourra être observée et contrôlée en continu, notamment dans sa phase finale.

L'association entre les brevets américains n°3,765,372 et n°4,742,798 ne rend pas intellectuellement évidente cette caractéristique en ce que la matière d'oeuvre traitée à savoir les oeufs des espèces élevés diffèrent grandement. En effet, le procédé proposé dans le document n°3,765,372 est un procédé d'élevage concernant des espèces marines dont les oeufs flottent et ne se déposent pas. Or, le dispositif décrit dans le document n°4,742,798 est spécifiquement conçu pour des oeufs de salmonidés qui ne flottent pas mais se déposent gravitairement sur les substrats prévus à cet effet et objet de l'invention décrite dans ce document. En conséquence, l'association de ces deux documents est non seulement non. évidente mais impossible pour l'homme de l'art qui connaît les caractéristiques des oeufs.

Contrairement à l'art antérieur représenté par le document américain n°US-A-4,742,798 dans lequel le dispositif est formé de substrats indépendants'les uns des autres, incapables de ce fait, de réaliser un déplacement global sans dommage à un moment précis des oeufs, le support amovible de l'invention assure ce déplacement qui peut avoir plusieurs fonctions.

Une autre caractéristique particulièrement intéressante du procédé de l'invention, consiste à retirer de l'incubateur le support amovible sur lequel sont fixés individuellement les oeufs de manière à interrompre le processus d'incubation à un moment déterminé et éviter de ce fait l'éclosion de larves dont l'embryogénèse aurait une durée trop longue.

En effet, la durée de l'embryogénèse à l'intérieur d'une opération d'incubation est plus ou moins variable selon les oeufs et les larves dont l'embryogénèse aurait une durée trop longue sont moins performantes. Leur élimination, dès avant l'éclosion, permet la sélection des sujets les plus performants et limite fortement la dispersion des tailles et conduit à des lots de production beaucoup plus homogènes, améliorant ainsi significativement la productivité aux plans quantitatifs et qualitatifs.

Une autre caractéristique particulièrement intéressante du procédé de l'invention consiste à placer ledit support amovible, après son retrait de l'incubateur, dans une zone de température différente de manière à provoquer un choc thermique sur les oeufs avant la première division cellulaire; l'application d'un choc thermique, différent suivant l'espèce concernée, permet d'obtenir des sujets triploïdes. Selon un exemple non limitatif de réalisation, pour Silurus Glanis, il s'agit d'un choc froid.

Le support amovible a pour avantage de permettre, à un même moment précis, le déplacement de la totalité des oeufs vers la zone de température différente et d'assurer l'exposition au choc thermique d'une durée identique pour l'ensemble des oeufs et chacun d'entre eux.

Ainsi, une fois définis, les trois paramètres variant en fonction de l'espèce élevée et traitée que sont le temps (ou moment) d'intervention, la température du choc thermique et la durée d'application dudit choc seront identiques pour tous les oeufs et chacun d'entre eux. Bien entendu, l'objectif de l'homogénéité du traitement en amont et durant la triploidie est de produire cent pour cent de sujets triploides, sans risques de diploïdie accidentelle qui reposerait le problème de pollution génétique et de gestion de l'élevage.

Ainsi, le support amovible présente plusieurs avantages selon l'embryogénèse désirée.

Dans le cadre d'une embryogénèse "normale", le support amovible permet :
- une bonne oxygénation,
- un contrôle visuel en continu en l'embryogénèse,
- une maîtrise de la durée de l'embryogénèse permettant une sélection efficace notamment de géniteurs.

Dans le cadre d'une embryogenèse soumise à choc thermique, ledit support amovible permet, en outre :
- l'homogénéisation du traitement thermique, et
- la maîtrise de l'ensemble des étapes de cette opération permettant là aussi, une sélection efficace des sujets les plus performants pour leur élevage.

L'invention concerne également le dispositif permettant de mettre en oeuvre ce procédé et remarquable en ce qu'il comporte un support amovible constitué par un treillis rigide formé d'une armature dont la forme générale s'adapte à la forme intérieure d'un incubateur de façon à pouvoir y être introduit, ledit treillis rigide permettant aux oeufs de s'y fixer individuellement au moment de leur introduction dans l'incubateur et pouvant être retiré de l'incubateur et remis.

Ce dispositif intervient à l'intérieur du cycle biologique bien refermé sur lui-même, comme un moyen déterminant, mais parmi d'autres moyens et procédés, de parfaire ce cycle et de permettre, du fait de ce cycle "parfait" et de son propre "rôle technique", d'accéder à une triploïdie "opératoire''. Autant le procédé que le dispositif sont, chacun de leur côté, la somme d'apports biologiques et techniques; l'invention est bien dans le résultat de cette somme mais également dans chacun de leurs éléments et leurs utilisations diversement associées.

Selon une caractéristique particulièrement avantageuse de l'invention, le treillis est formé par deux armatures circulaires liées entre elles par des montants verticaux de façon à ce que les centres des deux cercles soient sur le même axe formant un châssis rigide les montants verticaux et les cercles servant de support et de point d'attache à des fils s'entrecroisant et passant par chaque montant de façon à réaliser un treillage condensé à l'intérieur du châssis permettant aux oeufs de s'y fixer.

Le choix d'un fil comme support à la fixation des oeufs est particulièrement avantageux en ce qu'il permet une fixation sur une petite partie de la membrane, le reste de la surface sphérique de l'oeuf en assurant l'oxygénation.

Selon une caractéristique non limitative de l'invention, les fils constituant le treillage sont de diamètre inférieur au diamètre des oeufs auxquels ils servent de support.

Selon une autre caractéristique particulièrement avantageuse de l'invention, le châssis rigide ainsi que les fils sont en matériau imputrescible conduisant de façon optimale la température à laquelle ils ont été soumis-afin de garantir une très grande homogénéité dans le traitement thermique que l'on fait subir aux oeufs fixés au support.

Bien entendu, ledit treillis rigide est muni d'un organe de préhension lui permettant d'être retiré de l'incubateur, placé dans la zone de température différente et remis dans l'incubateur manuellement ou par un quelconque moyen de levage.

On comprend que le procédé et de dispositif qui viennent d'être ci-dessus décrits l'ont été en vue d'une divulgation plutôt que d'une limitation. Bien entendu, divers aménagements, modifications et améliorations pourront être apportés, sans pour autant sortir du cadre de l'invention défini par les revendications. Ainsi, le biotope auquel s'applique le procédé ne se limite pas à l'eau douce mais à toutes les situations où la vie aquatique est possible.

## Revendications

1. Procédé de reproduction et d'élevage en milieu aquatique sur la base d'oeufs susceptibles d'être fixés du type de celui comprenant les phases classiques suivantes :
- éclosion, résorption vésiculaire, différents stades d'élevage larvaire, élevage jusqu'au stade d'alevin ou de juvénile en écloserie,
- croissance et grossissement en bassins,
- passage de la phase alevin ou de la phase juvénile à la phase adulte,
- sélection des géniteurs, maturation,
- prélèvement des ovocytes et des spermatozoïdes,
- fécondation,
- embryogénèse en incubateur, **CARACTÉRISÉ EN CE QU'**il consiste à maintenir constamment la température de l'eau pendant l'ensemble des phases classiques d'élevage, à une même température unique la plus proche possible de l'optimum thermique biologique de l'espèce élevée.

2. Procédé de reproduction et d'élevage selon la revendication 1, **CARACTÉRISÉ EN CE QU'**il consiste à ne pas maintenir à une même température unique avant la phase d'élevage larvaire.

3. Procédé de reproduction et d'élevage selon la revendication 1 du type de celui où l'animal aquatique élevé est Silurus Glanis, **CARACTÉRISÉ EN CE QU'**il consiste à maintenir la température de l'eau pendant l'ensemble des phases classiques d'élevage, à une même température unique la plus proche possible de 26° celcius.

4. Procédé selon la revendication 1, **CARACTÉRISÉ EN CE QU'**il consiste à utiliser des bassins du type naturel et non aseptisé.

5. Procédé selon la revendication 1, **CARACTÉRISÉ EN CE QU'**il consiste à assurer l'alimentation en écloserie des larves, alevins et juvéniles sur la base d'aliments uniquement artificiels.

6. Procédé selon la revendication 1, **CARACTÉRISÉ EN CE QU'**il consiste à assurer l'alimentation en bassins sur la base d'une association d'aliments naturels et d'aliments artificiels.

7. Procédé selon l'une quelconque des revendications 1, 2 et 3, **CARACTÉRISÉ EN CE QU'**il QU'il consiste à placer dans l'incubateur, avant l'introduction des oeufs, un support amovible les accueillant sur des zones de réception permettant aux oeufs de s'y fixer individuellement au moment de leur introduction dans l'incubateur, lequel support permet, à un même moment précis, le déplacement de la totalité des oeufs.

8. Procédé selon la revendication 7, **CARACTÉRISÉ EN CE QU'**il consiste à retirer de l'incubateur le support amovible sur lequel sont fixés individuellement les oeufs de manière à interrompre le processus d'incubation à un moment déterminé et éviter de ce fait l'éclosion de larves dont l'embryogénèse aurait une durée trop longue.

9. Procédé selon la revendication 7, **CARACTÉRISÉ EN CE QU'**il consiste à placer ledit support amovible, après son retrait de l'incubateur, dans une zone de température différente de manière à provoquer un choc thermique sur les oeufs avant la première division cellulaire.

10. Dispositif permettant de mettre en oeuvre le procédé, selon l'une quelconque des revendications 7 à 9, comportant un incubateur et un support amovible constitué par un treillis rigide formé d'une armature dont la forme générale s'adapte à la forme intérieure de l'incubateur de façon à pouvoir y être introduit, ledit treillis rigide permettant aux oeufs de s'y fixer individuellement au moment de leur introduction dans l'incubateur et pouvant être retiré de l'incubateur et remis.

11. Dispositif selon la revendication 10, **CARACTÉRISÉ PAR LE FAIT QUE** le treillis est formé par deux armatures circulaires liées entre elles par des montants verticaux de façon à ce que les centres des deux cercles soient sur le même axe formant un châssis rigide les montants verticaux et les cercles servant de support et de point d'attache à des fils s'entrecroisant et passant par chaque montant de façon à réaliser un treillage condensé à l'intérieur du châssis permettant aux oeufs de s'y fixer.

12. Dispositif selon la revendication 11, **CARACTÉRISÉ PAR LE FAIT QUE** le châssis rigide ainsi que les fils sont en matériau imputrescible conduisant de façon optimale la température à laquelle ils ont été soumis afin de garantir une très grande homogénéité dans le traitement thermique que l'on fait subir aux oeufs fixés au support.

## Patentansprüche

1. Verfahren zur Reproduktion und Aufzucht im aquatischen Milieu auf der Basis von befestigungsfähigen Eiern vom Typ, welcher die folgenden klassischen Phasen beinhaltet:
- Erbrütung, vesikuläre Resorption, verschiedene Stadien der Larvenaufzucht, Aufzucht bis zum Stadium der Brut oder der Jungtiere in der Aufzuchtsanlage,
- Wachstum und Mast im Becken,
- Übergang von der Phase der Brut oder der Jungtierphase zur Erwachsenenphase,
- Auswahl der Erzeuger, Reifung,
- Entnahme der Oocyten und der Spermatocyten,
- Befruchtung,
- Embryogenese im Brutschrank,
**DADURCH GEKENNZEICHNET, DASS** die Temperatur des Wassers während der Gesamtheit der klassischen Aufzuchtsphasen konstant auf einer einheitlichen Temperatur gehalten wird, welche möglichst nahe am thermischen, biologischen Optimum der aufgezogenen Spezies liegt.

2. Verfahren zur Reproduktion und Aufzucht nach Anspruch 1, **DADURCH GEKENNZEICHNET, DASS** die Temperatur vor der Phase der Larvenaufzucht nicht konstant gehalten wird.

3. Verfahren zur Reproduktion und Aufzucht nach Anspruch 1 vom Typ, wobei das gezüchtete aquatische Tier Silurus Glanis ist, **DADURCH GEKENNZEICHNET, DASS** die Temperatur des Wassers während der Gesamtheit der klassischen Aufzuchtsphasen konstant auf einer einheitlichen Temperatur, welche möglichst nahe an 26° Celsius liegt, gehalten wird.

4. Verfahren nach Anspruch 1, **DADURCH GEKENNZEICHNET, DASS** natürliche und nicht keimfreie Becken benutzt werden.

5. Verfahren nach Anspruch 1, **DADURCH GEKENNZEICHNET, DASS** das Futter in der Aufzuchtsanlage der Larven, der Brut und der Jungtiere ausschließlich auf Basis von künstlichem Futtermittel gesichert ist.

6. Verfahren nach Anspruch 1, **DADURCH GEKENNZEICHNET, DASS** das Futter im Becken auf Basis eines Gemisches von natürlichen und künstlichen Futtermitteln gesichert ist.

7. Verfahren nach einem der Ansprüche 1, 2 und 3, **DADURCH GEKENNZEICHNET, DASS** in den Brutkasten, vor der Eingabe der Eier, ein beweglicher Träger eingesetzt wird, welcher diese an Rezeptionsstellen aufnimmt und welcher ermöglicht, dass die Eier sich im Moment der Eingabe in den Brutkasten einzeln festsetzen, dieser Träger ermöglicht, an einem bestimmten Zeitpunkt, die Bewegung der Gesamtheit der Eier.

8. Verfahren nach Anspruch 7, **DADURCH GEKENNZEICHNET, DASS** der bewegliche Träger, an welchem die Eier einzeln befestigt sind, aus dem Brutkasten entnommen wird, um den Brutprozess an einem bestimmten Zeitpunkt zu unterbrechen und um somit zu vermeiden, dass die Larvenheranreifung dessen Embryogenese eine zu lange Zeit beansprucht.

9. Verfahren nach Anspruch 7, **DADURCH GEKENNZEICHNET, DASS** der besagte, bewegliche Träger, nach der Entnahme aus dem Brutkasten, in eine andere Temperaturzone gebracht wird, wodurch vor der ersten zellulären Teilung ein thermischer Schock auf die Eier ausgewirkt wird.

10. Vorrichtung, welche die Umsetzung des Verfahrens nach einem der Ansprüche 7 bis 9 ermöglicht, welche einen Brutkasten und einen beweglichen, als steifes Gitter geformten Träger, welches aus einem Gerüst gebildet wird, dessen allgemeine Form sich der inneren Form des Brutkastens auf der Weise anpasst, dass es in diesen eingesetzt werden kann, beinhaltet, besagtes steifes Gitter ermöglicht den Eiern im Moment ihrer Eingabe in den Brutkasten sich einzeln festzusetzen und kann aus dem Brutkasten entnommen und wiedereingesetzt werden.

11. Vorrichtung nach dem Anspruch 10, DURCH DIE TATSACHE **GEKENNZEICHNET**, DASS das Gitter aus zwei kreisförmigen Gerüsten geformt ist, welche untereinander mit vertikalen Streben in solcher Weise verbunden sind, dass die Mittelpunkte der beiden Kreise auf der gleichen Achse liegen und somit ein steifes Gestell bilden; die vertikalen Streben und die Kreise dienen als Träger und Fixpunkte für Fäden, welche ineinanderverschlungen und auf solche Weise zwischen den Streben durchgeführt werden, dass ein dichtes Gitterwerk im Inneren des Gerüstes realisiert wird, welches die Fixierung der Eier ermöglicht.

12. Vorrichtung nach dem Anspruch 11, DURCH DIE TATSACHE **GEKENNZEICHNET**, DASS das feste Gestell sowie die Fäden aus einem unverweslichen Material bestehen, welches auf optimale Weise die Temperatur, auf welche sie gebracht wurden, leitet, um eine sehr gute Homogenität der thermischen Behandlung zu gewährleisten, welcher man den auf dem Träger befestigten Eiern aussetzt.

## Claims

1. A method for reproduction and rearing in an aquatic medium based on eggs able to be fixed, of the type comprising the following known phases:
- hatching, yolk absorption, various stages of larval rearing, raising to the stage of fry or juveniles in a hatchery.
- growth and fattening in pisciculture ponds
- passage from the fry stage or the juvenile stage to the adult stage,
- selection of spawning stock, maturing,
- ovocyte and spermatozoid collection,
- fertilisation
- embryogenesis in an incubator, **characterised in that** it consists of maintaining the water temperature constant during all the normal breeding stages, at a same and unique temperature the closest possible to the biological thermal optimum for the species being reared.

2. A method for reproduction and rearing according to claim 1, **characterised in that** it consists of not maintaining a same and unique temperature before the stage of larval rearing.

3. A method for reproduction and rearing according to claim 1 of the type in which the aquatic animal being bred is Silurus Glanis, **characterised in that** it consists of maintaining the water temperature, during all the normal rearing phases, at the same and unique temperature as close as possible to 26° Celsius.

4. A method according to claim 1, **characterised in that** it consists of using natural and non-sanitized pools.

5. A method according to claim 1, **characterised in that** it consists of ensuring feeding in a hatchery for larvae, fry and juveniles on the basis of artificial feeds only.

6. A method according to claim 1, **characterised in that** it consists of ensuring feeding in pools on the basis of an association of natural and artificial feeds.

7. A method according to any one of claims 1, 2 and 3, **characterised in that** it consists of placing in the incubator, before introducing eggs, a movable support for accepting them on reception zones enabling the eggs to fix themselves individually at the moment they are introduced into the incubator, said support enabling, at a precise and single moment, the displacement of the totality of the eggs.

8. A method according to claim 7, **characterised in that** it consists of withdrawing from the incubator the removable support on which the eggs are fixed individually, in such a way as to interrupt the incubation process at a determined moment and thus to avoid the hatching of larvae whose embryogenesis would have lasted for too long a time.

9. A method according to claim 7, **characterised in that** it consists of placing said removable support, after withdrawal from the incubator, in a different temperature zone so as to give a thermal shock to the eggs before the first cell division.

10. A device enabling operation of the method, according to any one of claims 7 to 9, comprising an incubator and a removable support constituted of a rigid trellis formed by a frame whose general shape adapts to the internal shape of the incubator in such a way as to be able to be introduced, said rigid trellis allowing the eggs to fix to it individually at the moment they are introduced into the incubator and being able to be withdrawn from the incubator and able to be reintroduced.

11. A device according to claim 10, **characterised in that** the trellis is formed by two circular frames linked together by vertical arms in such a way that the centres of the two circles are on the same axis forming a rigid chassis, the vertical uprights and the circles acting as support and attachment point for interlocking wires passing around each arm in such a way as to produce a dense mesh inside the chassis allowing the eggs to fix on it.

12. A device according to claim 11, **characterised in that** the rigid chassis as well as the wires are made in a rot-proof material conducting, in optimum fashion, the temperature to which they are submitted in order to guarantee very high homogeneity for the thermal treatment to which the eggs fixed to the support are submitted.
